Europäisches Patentamt

European Patent Office

Office européen des brevets

(11) Veröffentlichungsnummer: **0 302 852 B1**

(12) **EUROPÄISCHE PATENTSCHRIFT**

(45) Veröffentlichungstag der Patentschrift: **25.11.92**

(51) Int. Cl.⁵: **C02F 3/30**

(21) Anmeldenummer: **88890205.3**

(22) Anmeldetag: **05.08.88**

(54) **Verfahren zur Behandlung und Entsorgung von Gemengen aus Feststoffen und Flüssigkeiten.**

(30) Priorität: **06.08.87 AT 1995/87**

(43) Veröffentlichungstag der Anmeldung:
**08.02.89 Patentblatt 89/06**

(45) Bekanntmachung des Hinweises auf die
Patenterteilung:
**25.11.92 Patentblatt 92/48**

(84) Benannte Vertragsstaaten:
**AT BE CH DE ES FR GB GR IT LI LU NL SE**

(56) Entgegenhaltungen:
**DE-A- 2 504 412**
**FR-A- 2 240 890**
**FR-A- 2 336 353**
**FR-A- 2 541 669**

**A.M. BRUCE: "SEWAGE SLUDGE STABILISA-
TION AND DISINFECTION", 1984, Seiten
330-342, Ellis Horwood Ltd., Chichester GB;
Kapitel 17: D.C. BARTLETT: "Digester gas -
an energy resource"**

(73) Patentinhaber: **TECHNOAGRAR CONSULTING
AKTIENGESELLSCHAFT**
**Austrasse 52**
**FL-9490 Vaduz(LI)**

(72) Erfinder: **Lorinser, Martin**
**Reintalweg 24**
**A-8042 Graz(AT)**

(74) Vertreter: **Büchel, Kurt F., Dr. et al**
**Bergstrasse 297**
**FL-9495 Triesen(LI)**

# Beschreibung

Die Vorliegende Erfindung betrifft ein Verfahren nach dem Oberbegriff des Patentanspruches 1.

Die Behandlung und Entsorgung von Gemengen aus Feststoffen und Flüssigkeiten mit überwiegendem Flüssigkeitsanteil und organischen Komponenten, wie beispielsweise Molke, Gülle aus Massentierhaltungen u.dgl. wird, wenn überhaupt, heute durch anaerobe Vergärung im mesophilen bzw. thermophilen Bereich durchgeführt. In der Regel ist damit keine Totalentsorgung erreichbar. Die flüssige Komponente muß nach der anaeroben Behandlung noch einer aeroben Behandlung unterzogen werden, während der Schlamm bzw. Feststoffrückstand aus beiden Behandlungsstufen entweder mit erheblichen Kosten deponiert oder für eine wirtschaftliche Verwendung weiter behandelt werden muß. Oft ist gerade dieser Schlamm bzw. Feststoffrückstand Träger von Wertstoffen, welche während des anaeroben Verfahrens geschädigt oder in eine Form gebracht werden, welche nicht optimal ist.

So wird beispielsweise der organisch gebundene Stickstoff in eine Form gebracht, welche dem Handelsdünger sehr ähnlich ist, z.B. $NH_3$-Ammoniakstickstoff, welcher leicht auswaschbar ist. Nun wird gerade heute wieder Stickstoffdünger gesucht, welcher in organischer Form gebunden ist, wie z.B. die $NH_4$- oder $NH_4 + NO_3$-Gruppen, die eine echte Langzeitdüngung ohne Schädigung des Grundwassers sicherstellen.

Auch eine andere Verwertung des Stickstoffes wird angestrebt, nämlich als Proteinfuttermittel. Auch hierfür ist eine lange, anaerobe Behandlung abträglich, siehe r. Braun. Biogas-Methangärung organischer Abfallstoffe, Springer-Verlag, Wien 1982, Seiten 129 ff.

Letztlich ist die Entwässerung und beispielsweise Trocknung des anfallenden Schlammes aufwendig, vor allem wegen des hohen Wassergehaltes und der geringen Anteile an leicht kompostierbaren Inhaltsstoffen. Beim anaeroben Verfahren ist auch eine Gewisse Begrenzung des Feststoffanteiles notwendig, um Betriebsstörungen, z.B. durch Schwimmschlammbildung, auszuschalten. Am wirtschaftlichsten sind Substrate zu entsorgen, welche den Feststoff weitgehend in gelöster Form oder zumindest feinst verteilt enthalten. Hier ist die Abbauzeit kurz, die erforderliche Investition geringer und die Abbaurate über 90 % $BSB_5$. Die aerobe Nachbehandlung kann gering sein oder sogar entfallen, siehe Wilkie, A., Newell, P., 1981, The operation and economic assessment of full-scale anaerobic fixed bed reactions, 2nd Int. Symp. Anaerobic Digestion, Travemünde.

Das anfallende Biogas ist bei derartigen Substraten ebenfalls besser und enthält kaum Schadstoff, sodaß von einer aufwendigen Reinigung Abstand genommen werden kann, siehe Wilkie und Newell, loc. cit.

Die Verwertung des Biogases zur Wärmegewinnung ist in der Regel nicht ganzjährig möglich, sodaß der Erzeugung von Strom der Vorzug gegeben wird. Hiebei fällt eine Abwärme von bis zur 400° C Abgastemperatur an, welche nur zur Warmwassererzeugung eingesetzt werden kann. Das Warmwasser kann auch nicht ganzjährig verwendet werden.

Unter Berücksichtigung aller vorstehend aufgeführter Punkte wurde nun ein Verfahren konzipiert, das eine totale Entsorgung des anfallenden Gemenges sicherstellt, bei gleichzeitiger Ausnützung des Synergieeffektes zweier parallel laufender Einzelverfahren, nämlich einerseits der anaeroben Behandlung der Flüssigphase des Gemenges mit weitgehend nur gelöstem oder feinst verteiltem Feststoff, und anderseits der aeroben Behandlung des größeren Teiles des Feststoffes, welcher mechanisch aus dem Gemenge abgetrennt wurde, zusammen mit dem Schlamm aus den Flüssigphasen.

Gegenstand der vorliegenden Erfindung ist nun das eingangs genannte Verfahren mit den im Kennzeichen des Patentanspruches 1 angeführten Merkmalen.

Für die anaerobe Behandlung der Flüssigphase können alle Systeme, die heute bekannt sind, eingesetzt werden. Ebenfalls können für die aerobe Behandlung des Feststoffanteiles z.B. Schnellkompostierverfahren mit Kompostiertrommel oder ähnliche Verwendung finden.

Die Trennung des Gemenges in einen homogenen Flüssiganteil und einen Feststoffanteil kann z.B. mit Hilfe einer organischen Filterschicht, welche von Zeit zu Zeit erneuert wird, oder auch durch eine Siebbandpresse oder mit einem Dekanter erfolgen.

Für die Aufgabe in beispielsweise die Kompostiertrommel kann durch Beimischung von trockenem Endprodukt der aeroben Behandlung des Feststoffanteiles der gewünschte Wassergehalt erreicht werden, unter gleichzeitiger Einimpfung von Mikroorganismen in das Frischgut. Zur Beschleunigung der Fermentation wird Abwärme aus einem Gasmotor verwendet werden, welcher mit dem gebildeten Biogas betrieben wird und Strom erzeugt. Der Strom wird für den Betrieb der gesamten Anlage verwendet.

Ein weiterer Teil der Abwärme wird zweckmäßig zur Trocknung des Feststoffes, nachdem dieser die aerobe Behandlung durchlaufen hat, verwendet. Ein weiterer Teil der Abwärme, die als Gasmotorabwärme ziemlich wenig Sauerstoff enthält, wird zur direkten Vorwärmung des Flüssiganteils verwendet. Das Kohlendioxyd wird dabei mit dem Wasser Kohlensäure bilden und den pH-Wert der Flüssigkeit absenken, sodaß der anaerobe Abbau beschleunigt wird, oder beispielsweise mit dem $K_2O$ Kaliumkarbonat bilden, welches im Schlamm abgesetzt und dem Feststoff zugeführt wird. Der $K_2O$-Gehalt des Abwassers wird dadurch gesenkt. Durch die direkte Aufwärmung der Flüssigkeit durch Einperlen des heißen Abgases, zum Beispiel nach dem Venturi-Effekt, besteht auch keine Gefahr des Verlegens des Wärmetauschers durch Proteinkoagulation und Verlegen der Wärmetauscherrohre.

Durch eine zutreffende Anordnung der einzelnen Verfahrensteilschritte kann alle Abwärme aus der Stromerzeugung, welche ja nicht gleichmäßig sein wird, optimal genützt werden.

Der Schlamm, welcher aus der anaeroben Behandlung der Flüssigphase anfällt, wird vor der mechanischen Entwässerung in die Feststoffphase eingemischt. Dadurch läuft im Feststoff eine kurze anaerobe Phase ab, bei der verschiedene, aerob schwer zerlegbare Inhaltsstoffe im Feststoff, wie hoher Zucker, Mercaptane etc. aufgeschlossen und bei der anschließenden aeroben Phase leichter umgewandelt werden. Dadurch wird im Endprodukt keine unangenehme Geruchsentwicklung auftreten. Der Stickstoff, welcher im Faulschlamm enthalten ist, wird in der aeroben Phase organisch eingebunden und eine Grundwasserschädigung damit ausgeschlossen. Letztlich wird durch die hohe Abbaurate, welche in der abgesiebten Flüssigphase erreicht wird, die aerobe Nachbehandlung der Flüssigphase einfach und wirtschaflich. Geruchsentwicklungen sind bei einwandfreier Fahrweise ausgeschlossen.

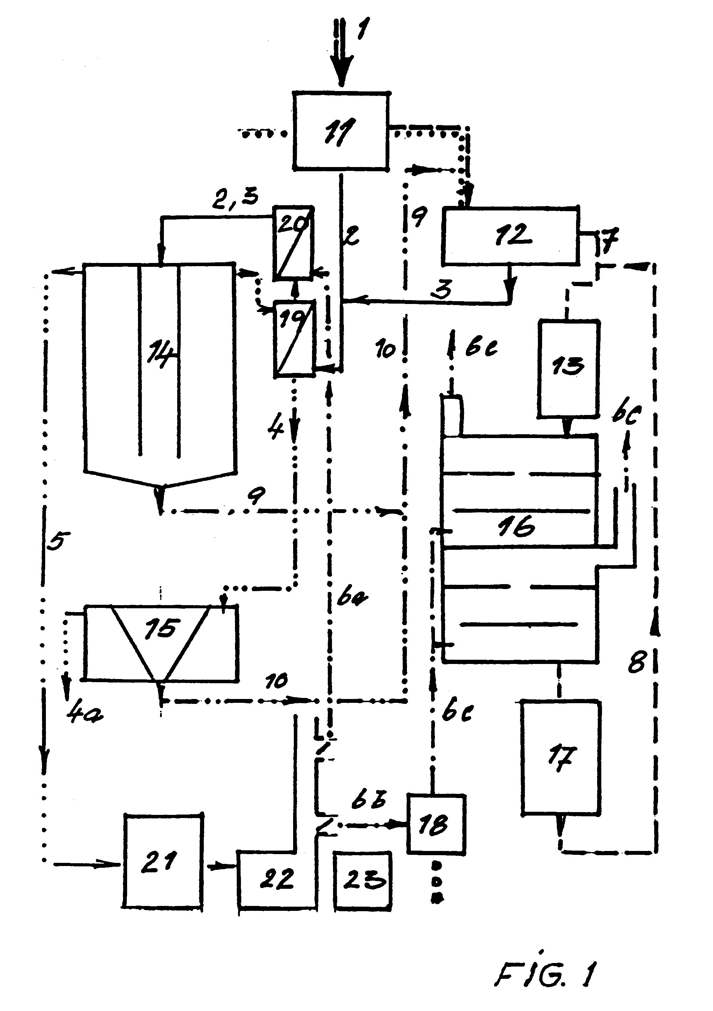

Anhand des beiliegenden Fließschemas Fig. 1 wird das Verfahren beispielhaft an der Entsorgung von Molke beschrieben, ist aber in keiner Weise darauf beschränkt.

Die Molke 1 wird in einem Festbettfilter 11 von zirka 50 g per l Feststoff auf zirka 20 g per l Feststoff gebracht. Als Filtermaterial werden beispielweise Heu, Spelzen oder Gras verwendet, welche chargenweise erneuert werden. Das Filtermaterial mit dem anfallenden Feststoffanteil, welcher auch anorganische Komponenten enthält, wird beispielsweise in einer Siebbandpresse 12 nochmals entwässert. Der Siebunterlauf 3 wird der Flüssigphase 2 zugeführt. Der Feststoffanteil 7 geht in den Vorratsbehälter 13. Vor der mechanischen Entwässerung wird dem Filterkuchen noch der Schlamm aus der anaeroben 9 und der aeroben Behandlung 10 der Flüssigphase der Molke zugegeben.

Der Faulschlamm aus der anaeroben Behandlung 9 verursacht im Aufgabehälter 13 eine kurze, anaerobe Phase. Der Feststoff wird dann zum Beispiel einem vertikalen Rotteturm 16 aufgegeben.

Hier wird in den oberen Etagen ein aerober Rotteprozess ablaufen, wobei ein Gemenge von Luft und Motorabgas 6c eingedrückt wird, um den aeroben Prozess zu beschleunigen. Dieser ist nach 12 bis $16^h$ abgeschlossen. In den unteren Etagen des Rotteturms wird eine wesentlich größere Menge von Luft und Motorabgas durchgedrückt, um den Feststoff auf zirka 15 % Restwassergehalt zur trocknen. Das Endprodukt (im Sammelbehälter 17) ist ein Futtermittel mit zirka 20 - 25 % Protein und mineralischen Spurenelementen, welches sich hervorragend für Rindermast eignet. Mit 15 % Wassergehalt ist es auch lagerbar. Für die Vermarktung kann es granuliert werden. Ein Teil des erhaltenen Produktes wird als Rückgut 8 dem Feststoffanteil 7 zur Feuchteeinstellung und zum Animpfen zugesetzt.

Die Flüssigphase der Molke 2 zusammen mit dem Siebunterlauf 3 werden der anaeroben Ausfaulung 14 zugeführt. Da dieser Prozess heute in der Regel thermophil abläuft, werden Flüssigphase 2 und Siebunterlauf 3, welche im Jahresmittel mit 15° C anfallen, in zwei Wärmetauschern 19, 20 auf zirka 55° C gebracht. Im ersten Wärmetauscher 19 wird das ablaufende Wasser der Flüssigphase 4 auf zirka 22 °C gekühlt und im Gegenstrom Flüssigphase 2 und Siebunterlauf 3 auf zirka 45° C erwärmt. Im zweiten Wärmetauscher werden dann mittels eines Teilstromes 6a des Motorgases die Flüssigphase 2 und der Siebunterlauf 3 entweder indirekt oder direkt durch Einperlen des Motorabgases auf 55° C erwärmt. Beim direkten Einperlen müßte anschließend, beispielsweise mittels Zyklonen, eine Entlüftung der Flüssigphase durchgeführt werden, bevor sie in den Reaktor 14 gebracht wird.

Das ablaufende Wasser 4 wird einer kurzen, aeroben Behandlung 15 zum Beispiel durch Bodenbelüftung ausgesetzt, geht durch ein Absetzbecken und kann über Leitung 4a jedem Vorfluter zugeleitet werden. Das produzierte Biogas 5 wird in einem Zwischentank 21 kurz gespeichert. Auf Grund der Vorbehandlung der Molke ist eine Reinigung des Biogases vor seiner Verwendung in einem Gasmotor nicht erforderlich.

Ein Elektroblock, bestehend aus einem Gasmotor 22 und einem Generator 23, erzeugt Strom für die ganze Anlage und kann auch Strom nach außen liefern. Das Motorabgas, welches mit zirka 400° C Abgastemperatur anfällt, reicht aus, um einerseits dir Vorwärmung der Flüssigphase 2, 3 sicherzustellen und anderseits - mit Luft vermischt - über das Gebläse 18 die Wärme für die mikrobiologische Phase des Rotteturmes sowie für die anschließende Trocknungsphase zu liefern.

Bei Feststoff-Flüssigkeits-Gemengen mit hohem Stickstoffgehalt, wie Schweinegülle, kann im ablaufenden Wasser aus der anaeroben Phase ein so hoher Stickstoffgehalt in Form von $NH_4$ und

NH₃ auftreten, daß dieser in der nachfolgenden aeroben Phase vor allem wegen des Mangels an Kohlenstoff nicht vollständig abgebaut werden kann.

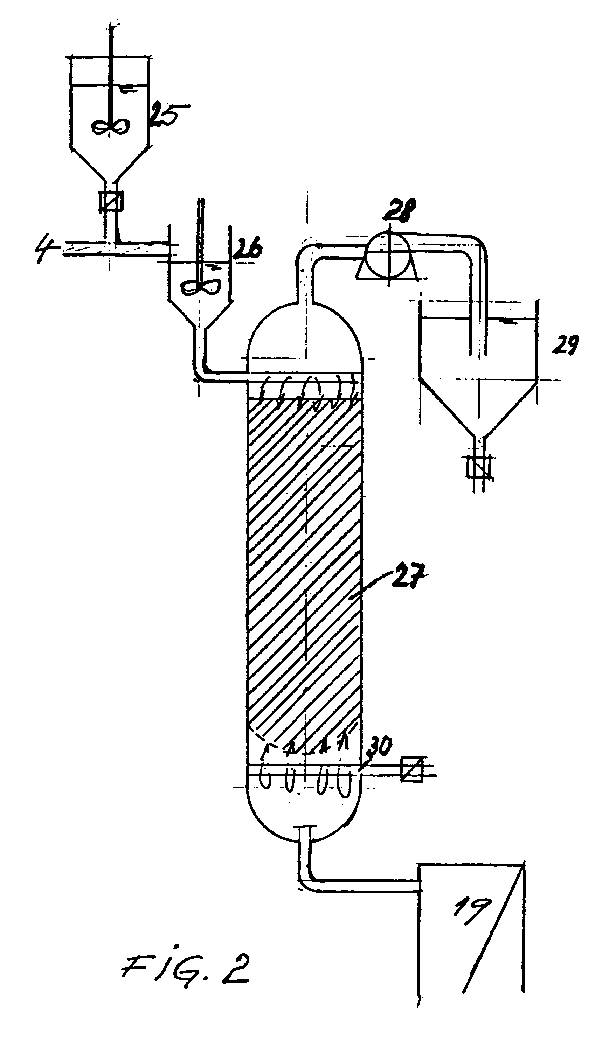

Gemäß einer vorteilhaften Ausführungsform der Erfindung kann daher zwischen der anaeroben Phase und der aeroben Phase eine zusätzliche Behandlung der Flüssigphase 4 aus der anaeroben Ausfaulung 14 eingeschoben werden, die in Fig. 2 dargestellt ist.

In der ablaufenden Flüssigphase 4 wird beispielsweise mittels Kalkmilch, welche aus einem Behälter 25 zudosiert wird, der pH-Wert auf 9 angehoben. Bei diesem pH-Wert und der Temperatur von 50° C liegt ein Großteil des Stickstoffs in Form von Ammoniak vor. Das aus dem Mischer 26 ablaufende Wasser wird einer Füllkörperkolonne 27 aufgegeben. Am Kopf der Füllkörperkolone 27 wird mittels eines Saugzuggebläses 28 in der Kolone 27 ein leichter Unterdruck von zirka 100 mm Wassersäule erzeugt. Dadurch dampft das Ammoniak aus dem ablaufenden Wasser 4 aus und wird mittels des Saugzuggebläses 28 beispielsweise in eine Schwefelsäurelösung 29 gedrückt. Das Ammoniak reagiert mit der Schwefelsäure zu Ammonsulfat und fällt aus.

Zur Absenkung der pH-Wertes in dem ablaufenden Wasser 4 wird am unteren Teil der Kolonne 27 über Rohrschlitze 30 so viel Motorabgas zugeführt, daß durch die darin enthaltene Kohlensäure der pH-Wert auf zirka 7,5 abgesenkt wird. Aus dem Sumpf der Kolonne 27 geht das ablaufende Wasser 4 dann in den Wärmetauscher 19 (siehe Fig. 1) und anschließend in die aerobe Phase 15.

## Patentansprüche

1. Verfahren zur getrennten Behandlung und Entsorgung von Gemengen aus Feststoffen und Flüssigkeiten mit organischen Komponenten, wobei das Gemenge durch mechanische Trennung in eine Flüssigphase mit einem niedrigen Feststoffgehalt in feinstverteilter Form und einen Feststoffanteil mit einem Wassergehalt von 45 bis 70 Gewichtsprozent aufgespalten wird, worauf die Flüssigphase einem anaeroben, der Feststoffanteil einem aeroben Fermentationsvorgang unterworfen werden, wobei gegebenenfalls der aus der anaeroben Behandlung der Flüssigphase resultierende Schlamm mit dem Feststoffanteil vermischt wird, dadurch gekennzeichnet, dass vor der aeroben Weiterbehandlung der Feststoffanteil einer kurzzeitigen anaeroben Behandlung unterworfen, das während des anaeroben Fermentationsvorganges der Flüssigphase gewonnene Biogas in einem Gasmotor verbrannt und das Abgas wenigstens teilweise zur Erwärmung und/oder zur Reduktion des pH-Wertes wenigstens einer der Flüssigphasen (2,3,4) und/oder des Feststoffanteils (7) vor dessen aerober Behandlung verwendet wird, während die Motorabwärme gegebenenfalls teilweise zur Trocknung des Endproduktes der aeroben Behandlung verwendet wird.

2. Verfahren nach Anspruch 1, dadurch gekennzeichnet, daß zur mechanischen Trennung des Gemenges ein organisches Filtermaterial, beispielsweise Spelzen oder Gras, eingesetzt wird.

3. Verfahren nach Anspruch 1, dadurch gekennzeichnet, daß zur mechanischen Trennung des Gemenges ein anorganisches Filtermaterial, beispielsweise Urgesteinsmehl, eingesetzt wird.

4. Verfahren nach einem der Ansprüche 1 bis 3, dadurch gekennzeichnet, daß bei hohen Stickstoffgehalten der aus der anaeroben Behandlung ablaufenden Flüssigphase vorerst der pH-Wert dieser Flüssigphase, beispielsweise mittels Kalkmehl auf 9 - 10 angehoben wird und danach das entstehende Ammoniak in einer Rieselkolonne ausgedampft wird und der Ammoniakdampf beispielsweise in ein Schwefelsäurebad eingeleitet wird, worin das Ammoniak als Ammoniumsulfat abgeschieden wird.

## Claims

1. A method of separately treating and disposing of mixtures of solids and liquids with organic components, wherein the mixture is divided up by mechanical separation into a liquid phase with a low solids content in very finely distributed form and a solids component with a water content of from 45 to 70% by weight, whereupon the liquid phase is subjected to an anaerobic fermentation operation and the solids component is subjected to an aerobic fermentation operation, wherein optionally the slurry resulting from the anaerobic treatment of the liquid phase is mixed with the solids component, characterised in that prior to the further aerobic treatment the solids component is subjected to a brief anaerobic treatment, the biogas produced during the anaerobic fermentation operation on the liquid phase is burnt in a gas engine and the exhaust gas is at least partially used for heating and/or for reducing the pH-value of at least one of the liquid phases (2, 3, 4) and/or the solids component (7) prior to the aerobic treatment thereof while the waste engine heat is possibly partially used for drying the end product of the aerobic treatment.

2. A method according to claim 1 characterised in that an organic filter material, for example husks or glumes or grass, is used for mechanical separation of the mixture.

3. A method according to claim 1 characterised in that an inorganic filter material, for example primitive rock dust, is used for mechanical separation of the mixture.

4. A method according to one of claims 1 to 3 characterised in that, with high nitrogen contents in the liquid phase issuing from the anaerobic treatment, the pH-value of that liquid phase is firstly raised to 9 - 10, for example by means of lime dust, and thereafter the resulting ammonia is evaporated in a trickle column and the ammonia vapour is introduced for example into a sulphuric acid bath wherein the ammonia is deposited as ammonium sulphate.

**Revendications**

1. Procédé de traitement séparé et d'élimination de mélanges de substances solides et de liquides avec des composants organiques, où le mélange est craquée par séparation mécanique en donnant une phase liquide avec une faible teneur en solides, sous forme très finement répartie, et une proportion en phase solide, avec une teneur en eau allant de 45 à 70 pourcent en poids, à la suite de quoi la phase liquide est soumise à un processus de fermentation anaérobie et la proportion en substance solide est soumise à un processus de fermentation aérobie, où, le cas échéant, la boue résultant du traitement anaérobie de la phase liquide est mélangée à la proportion en phase solide, caractérisé en ce qu'avant de continuer le traitement aérobie, la proportion en substance solide est soumise brièvement à un traitement anaérobie, le gaz biologique obtenu pendant le processus de fermentation anaérobie de la phase liquide étant brûlé dans un moteur et les gaz d'échappement étant utilisés au moins partiellement pour réchauffer et/ou réduire la valeur du pH d'au moins l'une des phases liquides (2,3,4) et/ou de la proportion en phase solide (7), avant sont traitement aérobie, tandis que la chaleur évacuée par le moteur est utilisée, le cas échéant, partiellement, pour sécher le produit final du traitement aérobie.

2. Procédé selon la revendication 1, caractérisé en ce qu'un matériau filtrant organique, par exemple des glumes ou de l'herbe, est utilisé pour opérer la séparation mécanique du mélange.

3. Procédé selon la revendication 1, caractérisé en ce qu'un matériau filtrant anorganique, par exemple de la farine de roche primitive, est utilisé pour opérer la séparation mécanique du mélange.

4. Procédé selon l'une des revendication 1 à 3, caractérisé en ce que, dans le cas de fortes teneurs en azote de la phase liquide s'écoulant du traitement anaérobie, on commence par augmenter à 9 à 10 la valeur de pH de cette phase liquide, par exemple au moyen de farine de chaux, et qu'ensuite on vaporise l'ammoniac produit dans une colonne garnie de lavage, la vapeur d'ammoniac étant par exemple dirigée dans un bain d'acide sulfurique, où l'ammoniac est précipité en sulfate d'ammonium.

FIG. 1

FIG. 2